# EUROPÄISCHE PATENTSCHRIFT

(11) **EP 0 777 468 B1**
(45) Veröffentlichungstag und Bekanntmachung des Hinweises auf die Patenterteilung: **29.09.1999**
(21) Anmeldenummer: 95929855.5
(22) Anmeldetag: 12.08.1995
(51) Int. Cl.: A61K 9/70

(54) **MEDIZINISCHER HAFTKLEBER**
MEDICAL CONTACT ADHESIVE
ADHESIF DE CONTACT A USAGE MEDICAL

(30) Priorität: 23.08.1994 DE 4429791
(43) Veröffentlichungstag der Anmeldung: 11.06.1997
(73) Patentinhaber: LTS LOHMANN Therapie-Systeme GmbH, 56567 Neuwied (DE)
(72) Erfinder: MÜLLER, Walter, D-56564 Neuwied (DE)
(74) Vertreter: Flaccus, Rolf-Dieter, Dr.
(86) Internationale Anmeldenummer: EP9503200
(87) Internationale Veröffentlichungsnummer: WO9605813

(56) Entgegenhaltungen:
- DE-A- 3 643 987
- DE-C- 4 301 783

## Beschreibung

Die Erfindung bezieht sich auf einen für Wundschnellverbände geeigneten, durch Metallchelate vernetzbaren medizinischen Haftkleber ohne hautreizende und ohne allergene Wirkung, dessen Klebrigkeit bei Aufnahme von Feuchtigkeit deutlich ansteigt, sowie dessen Verwendung.

Haftklebende Materialien werden in der medizinischen Versorgung des Menschen seit langer Zeit für die vielfältigsten Zwecke eingesetzt. Beispielhaft seien genannt Wundschnellverbände , Fixierpflaster, selbstklebend ausgerüstete Elektroden, orthopädische Tapes und speziell aus neuerer Zeit auch wirkstoffhaltige Pflaster.

Da diese Haftkleber mit der Haut des Menschen in Kontakt kommen, besteht eine wesentliche Forderung darin, daß sie über keine primär hautreizenden Eigenschaften und kein allergenes Potential verfügen dürfen. Das allergene Potential ist ein spezielles Problem von Haftklebern auf der Basis von natürlichem Kautschuk oder vergleichbaren synthetischen Polymeren. Da diese primär nichtklebend sind, müssen sogenannte Klebrigmacher zugesetzt werden, die meistens Derivate von Baumharzen, d.h. Kollophoniumderivate sind. Diese Kollophoniumderivate werden normalerweise als Gemisch eingesetzt und rufen zumindest bei wiederholtem Gebrauch bei einer nicht zu vernachlässigenden Anzahl von Menschen zum Teil heftige allergische Reaktionen hervor.

Moderne Haftkleber auf der Basis von synthetischen Polyacrylatpolymeren und Silikonen sind auch ohne den Zusatz von Klebrigmachern allein aufgrund ihrer Molekulargewichts-Verteilung und der verwendeten Monomere selbstklebend und verfügen über ein sehr geringes allergenes Potential. Solche Haftkleber werden als hypoallergen bezeichnet. Obwohl mit diesen Haftklebern das Problem der Hautverträglichkeit als gelöst bezeichnet werden kann, erfüllen auch diese nicht alle Anforderungen, die an medizinische Hochleistungshaftkleber zu stellen sind. Ein Problem besteht darin, daß medizinische Haftkleber auf der menschlichen Haut eingesetzt werden und diese starken interindividuellen Zustandsänderungen unterliegt. Beispielsweise ändert die Haut als biologisches Gewebe ihre Substrateigenschaften in Abhängigkeit von den jeweiligen Lebens- und/oder Umweltbedingungen. Ein Hauptproblem bildet dabei das Schwitzen der Haut bei körperlicher Belastung und/oder Krankheitszuständen bzw. bei hohen Umgebungstemperaturen. Ein medizinischer Haftkleber muß aber auch unter diesen Bedingungen eine sichere Haftung auf der Haut garantieren. Eine Möglichkeit, dies zu erreichen, ist es, den Haftkleber möglichst aggressiv klebend zu machen. Erkauft wird dies allerdings dadurch, daß der Kleber dann sehr weich ist und das unerwünschte Zurückbleiben von Kleberresten auf der Haut praktisch nicht vermieden werden kann.

Ein idealer Haftkleber sollte deshalb über eine Mindestklebrigkeit verfügen, die erst unter Einwirkung von Feuchtigkeit in eine aggressivere Klebrigkeit übergeht. Bei Polyacrylatklebern wird die Klebrigkeit von der Art der für die Synthese verwendeten Monomere und vor allem durch sein mittleres Molekulargewicht sowie die Molekulargewichtsverteilung bestimmt. Niedrige Molekulargewichte führen dabei zu einem weichen und aggressiven Kleber.

Der Erfindung liegt die Aufgabe zugrunde, einen Haftkleber anzugeben, der bei ausreichender Mindestklebrigkeit unter Einwirkung von Feuchtigkeit seine Molekulargewichtsverteilung ändert und dabei in einen Zustand aggressiverer Klebrigkeit übergeht.

Die Lösung dieser Aufgabe gelingt bei einem Haftkleber auf Basis von Polyacrylaten mit der Erfindung durch eine Zusammensetzung entsprechend dem Kennzeichnungsteil von Anspruch 1.

Weitere Ausgestaltungen der Zusammensetzung des medizinischen Haftklebers sind entsprechend den Unteransprüchen vorgesehen. Dieser eignet sich zur Verwendung bei der Herstellung von Wundschnellverbänden, Fixierpflastern oder selbstklebenden Elektroden sowie von wirkstoffhaltigen Pflastern.

Zwar werden Polymermassen auf Acrylatbasis mit einpolymerisierter (Meth)Acrylsäure unter einer Vielzahl von anderen Materialien als Bestandteil einer haftklebenden Reservoirschicht für die Verabreichung von Galanthamin in der DE 43 01 783 C1 der Anmelderin genannt und in einer tabellarischen Zusammenstellung von Versuchen zur Galanthamin-Penetration werden als Beispiele Galanthamin enthaltende Massen aus Acrylatcopolymeren von 2-Ethylhexylacrylat, Vinylacetat uns Acrylsäure, Octansäure als Penetrationsförderer, Isopropylmyristat als Weichmacher sowie einem Methacrylatcopolymeren auf der Basis von Dimethlaminomethacrylat und neutralen Methacrylsäureestern angegeben. Aus dieser speziellen Entwicklung zur Verbesserung der Galanthaminapplikation über die integere Haut konnte jedoch keinesfalls auf ein besonders brauchbares Verhalten insbesondere im Bereich der Wundversorgung erfindungsgemäß präzisierter Massen geschlossen werden, deren Zusammensetzung zur Reservoirschicht der Penetrationsversuche eine gewisse Verwandtschaft zeigt.

Beim erfindungsgemäßen Kleber kommt es zu einer Wechselwirkung zwischen den sauren Carboxylgruppen des Polyacrylatpolymers und dem basische Gruppen enthaltenden Polymer. Der Kleber liegt damit in einem Zustand von Vernetzung vor, die eine Molekulargewichtserhöhung zur Folge hat.

Vernetzungsreaktionen spielen bei den Polyacrylatklebern eine große Rolle. Wenn der Kleber über Hydroxyl- oder Carboxylgruppen verfügt, ist es möglich, durch den Zusatz von z.B. Metallchelaten den Kleber während der Entfernung der Lösemittel zu vernetzen. Man spricht dann von selbstvernetzenden Klebern. Der Vorteil dieser Kleber ist es, daß man Kleberlösungen erhält, die bei einem hohen Feststoffanteil über eine geringe Viskosität verfügen. Die endgültige Molekulargewichtsverteilung stellt sich bei diesen Klebern erst nach dem Entfernen der Lösemittel ein.

Während diese Art der Vernetzung irreversibel ist, bleibt eine Vernetzung durch Wechselwirkung von basischen und sauren funktionellen Gruppen voll reversibel. Weil dabei die Vernetzung nicht durch chemische Bindung, sondern lediglich über elektrostatische Kräfte bewirkt wird, kann sie beispielsweise durch Wasser infolge seiner hohen Dielektrizitätskonstante geschwächt werden. Der Kleber verhält sich dabei so, als ob sich seine Molekulargewichtsverteilung ändert. Er wird weicher, beginnt aggresiver zu kleben und paßt sich somit den jeweiligen Erfordernissen an. Diese Art der Vernetzung kann deshalb als "dynamische Vernetzung" bezeichnet werden. Weil die Haut in Abhängigkeit von den jeweiligen Gegebenheiten mehr oder weniger Feuchtigkeit abgibt, tritt dieser Effekt auch bei dem auf die Haut applizierten erfindungsgemäß aufgebauten Kleber auf.

Um eine gewisse Mindest- oder Grundklebrigkeit zu erreichen, ist dem Haftkleber erfindungsgemäß ein Weichmacher beigemischt. Die Menge dieses Weichmachers richtet sich nach den erwünschten Klebeeigenschaften des selbstklebenden Polyacrylats und dem Vernetzungsgrad durch das zugesetzte Polymer mit basischen Gruppen. Bei niedrigem Molekulargewicht der selbstklebenden Polyacrylate muß zusätzlich eine chemische Quervernetzung dieser Polymere vorgenommen werden. Dies wird erreicht durch Zusatz von Metallchelaten, z.B. Aluminiumacetylacetonat, oder anderen geeigneten Substanzen.

Als Weichmacher eignen sich im Prinzip alle niedrigmolekularen Verbindungen, die mit den Polymeren kompatibel sind. Dabei ist zu berücksichtigen, daß diese Weichmacher über eine gute Hautverträglichkeit verfügen. Als besonders geeignet haben sich Fettsaäurederivate des Glycerins sowie Triacetin erwiesen.

Es sind zwar auch andere Kleber bekannt, die bei Anwesenheit von Wasser ihre Klebeeigenschaften ändern. Erinnert sei zum Beispiel an einen Briefmarkenkleber, der erst beim Anfeuchten seine klebrigen Eigenschaften entwickelt. Diese Art von Klebern hat allerdings den entscheidenden Nachteil, daß sie kaum feuchtigkeitsresistent sind und sich bei Überangebot von Wasser auflösen.

Ein Haftkleber im Sinne der Erfindung ist im Gegensatz dazu sehr wasserresistent. In Wasser mit neutralem pH-Wert ist er praktisch unlöslich. Weiter verfügt er über hervorragende Klebeeigenschaften auch unter schwierigsten Bedingungen. Er ist überall einsetzbar, wo ein gutes und zuverlässiges Kleben über längere Zeit auf der Haut gefordert ist. Besonders vorteilhaft ist er anzuwenden zur Fixierung von Kanülen und Kathedern, bei Elektroden zur Registrierung von Biosignalen, Allergietestpflastern, Tapes für orthopädische Zwecke und nicht zuletzt für wirkstoffhaltige Pflaster.

### Beispiel:

Herstellung eines selbstklebenden Films
- 209 g: selbstklebendes, Carboxylgruppen enthaltendes Polyacrylat (Durotak 901-1051, 52% g/g in Ethylacetat/n-Heptan-Gemisch)
- 4,8 g: Polyacrylat mit basischen Aminogruppen (50% g/g in Ethanol) (Eudragit E 100)
- 8 g: Triacetin
- 15 g: Ethanol und
- 3,15 g: Aluminiumacetylacetonat (4% g/g in Ethylacetat)
werden zusammengerührt und als 350 µm dicker Film auf eine silikonisierte Polyesterfolie beschichtet. Der Film wird 30 Minuten bei 50°C getrocknet und hat dann ein Flächengewicht von 120 g/m².

Der getrocknete Film kann je nach Verwendungszweck mit textilen Geweben, Gewirken oder Vliesen kaschiert werden.

## Patentansprüche

1. Medizinischer Haftkleber auf Basis von Polyacrylaten mit im Applikationszustand ausreichender Mindestklebrigkeit, dessen Klebeigenschaft bei Einwirkung von Feuchtigkeit aufgrund reversibler Vernetzung zunimmt, dadurch gekennzeichnet, daß er folgende Komponenten enthält:
a) ein selbstklebendes Polyacrylatcopolymer mit mindestens 3 Molprozenten einpolymerisierter Acryl- oder Methacrylsäure,
b) ein basische Aminogruppen enthaltendes Polymer,
c) einen durch Triacetin oder Glycerinfettsäureester gebildeten Weichmacher,
d) Metallchelat, insb. Aluminiumacetylacetonat als Vernetzungsmittel.

2. Medizinischer Haftkleber nach Anspruch 1, dadurch gekennzeichnet, daß er sich nach Entfernen von Lösemittel enthält:
a) 75-94 Gew.-% selbstklebendes Polyacrylatcopolymer mit mindestens 3 Molprozenten einpolymerisierter Acryl- oder Methacrylsäure,
b) 1-20 Gew.-% Polymer mit basischen Aminogruppen und
c) 5-20 Gew.-% Weichmacher

3. Medizinischer Haftkleber nach Anspruch 1 oder 2, dadurch gekennzeichnet, daß das Polymer mit basischen Aminogruppen ein Polyacrylat- oder Polymethacrylatcopolymer ist und einpolymerisiert Aminoalkoholester der Acryl- oder Methacrylsäure enthält.

4. Medizinischer Haftkleber nach Anspruch 1 oder 2, dadurch gekennzeichnet, daß das Polymer mit basischen Aminogruppen Dimethylaminoethylmethacrylat und neutrale Methacrylsäureester enthält.

5. Verwendung eines Haftklebers gemäß einem oder mehreren der vorangegangenen Ansprüche zur Herstellung von Wundschnellverbänden, Fixierpflastern oder selbstklebenden Elektroden.

6. Verwendung eines Haftklebers gemäß einem oder mehreren der Ansprüche 1 bis 4 zur Herstellung von wirkstoffhaltigen Pflastern.

## Claims

1. Medical pressure-sensitive adhesive based on polyacrylates, having a minimal degree of tackiness in the state of application and whose tackiness increases upon action of moisture due to reversible crosslinking, characterized in that it comprises the following components:
a) a self-adhesive polyacrylate copolymer containing at least 3 mol-per cent of copolymerized acrylic or methacrylic acid
b) a polymer containing basic amino groups
c) a plasticizer formed by triacetin or glycerol fatty acid ester
d) metal chelate, especially aluminium acetylacetonate, as crosslinking agent.

2. Medical pressure-sensitive adhesive according to Claim 1, characterized in that following removal of solvent it comprises:
a) 75 - 94 % by weight of self-adhesive polyacrylate copolymer containing at least 3 mol-per cent of copolymerized acrylic or methacrylic acid,
b) 1 - 20 % by weight of polymer containing basic amino groups, and
c) 5 - 20 % by weight of plasticizer.

3. Medical pressure-sensitive adhesive according to Claim 1 or 2, characterized in that the polymer containing basic amino groups is a polyacrylate or polymethacrylate copolymer and contains, incorporated by polymerization, amino alcohol esters of acrylic or methacrylic acid.

4. Medical pressure-sensitive adhesive according to Claim 1 or 2, characterized in that the polymer containing basic amino groups comprises dimethylaminoethyl methacrylate and neutral methacrylic esters.

5. Use of a pressure-sensitive adhesive according to one or more of the preceding claims for producing quick dressings, fixing plasters or self-adhesive electrodes.

6. Use of a pressure-sensitive adhesive according to one or more of claims 1 to 4 for producing medicated plasters.

## Revendications

1. Adhésif de contact à usage médical à base de polyacrylates avec une adhésivité minimale suffisante à l'état d'application, dont la propriété d'adhésivité augmente lors de l'action de l'humidité sur base d'une réticulation réversible, caractérisé en ce qu'il contient les composants suivants :
a) un copolymère de polyacrylate auto-adhésif avec au moins 3 % molaires d'acide acrylique ou méthacrylique, incorporés par polymérisation,
b) un polymère contenant des groupes amino basiques,
c) un agent plastifiant formé de triacétine ou d'ester d'acide gras de glycérine,
d) un chélate métallique, en particulier l'acétylacétonate d'aluminium, à titre d'agent de réticulation.

2. Adhésif de contact à usage médical selon la revendication 1, caractérisé en ce qu'il contient, après l'élimination des solvants :
a) 75 à 94 % en poids de copolymère de polyacrylate auto-adhésif avec au moins 3 % molaires d'acide acrylique ou méthacrylique incorporés par polymérisation,
b) 1 à 20 % en poids de polymère avec des groupes amino basiques et
c) 5 à 20 % en poids d'agent plastifiant.

3. Adhésif de contact à usage médical selon la revendication 1 ou 2, caractérisé en ce que le polymère avec des groupes amino basiques est un copolymère de polyacrylate ou de polyméthacrylate et contient des esters d'amino-alcool de l'acide acrylique ou méthacrylique, incorporés par polymérisation.

4. Adhésif de contact à usage médical selon la revendication 1 ou 2, caractérisé en ce que le polymère avec des groupes amino basiques contient du méthacrylate diméthylaminoéthylique et des esters neutres de l'acide méthacrylique.

5. Utilisation d'un adhésif de contact selon une ou plusieurs des revendications précédentes pour la préparation de pansements rapides, de sparadraps de fixation ou d'électrodes auto-adhésives.

6. Utilisation d'un adhésif de contact selon une ou plusieurs des revendications 1 à 4 pour la préparation d'emplâtres contenant des principes actifs.
